# EUROPEAN PATENT SPECIFICATION

(11) **EP 4 452 385 B1**
(45) Date of publication and mention of the grant of the patent: **11.02.2026**
(21) Application number: 22851222.4
(22) Date of filing: 20.12.2022
(51) Int. Cl.: A61M 31/00

(54) **URINARY PLACEMENT CATHETER FOR INTRAVESICAL DEVICES**
URINPOSITIONIERUNGSKATHETER FÜR INTRAVESIKALE VORRICHTUNGEN
CATHÉTER DE PLACEMENT URINAIRE POUR DISPOSITIFS INTRAVÉSICAUX

(30) Priority: 21.12.2021 US 202163292063 P
(43) Date of publication of application: 30.10.2024
(73) Proprietor: TARIS Biomedical LLC, Lexington, Massachusetts 02421 (US)
(72) Inventor: DIUBALDI, Anthony, Lexington, Massachusetts 02421 (US); FLEMING, James, Lexington, Massachusetts 02421 (US); HO DUC, Hong Linh, Lexington, Massachusetts 02421 (US); JOHNSON, Wayne, Louisville, Kentucky 40229 (US); LEWIS, Mark, Lexington, Massachusetts 02421 (US); MELLO, Jason, Tyngsboro, Massachusetts 01879 (US)
(74) Representative: Carpmaels & Ransford LLP
(86) International application number: PCT/US2022/053460
(87) International publication number: WO 2023/122058

(56) References cited:
- WO-A1-00/66213
- US-A- 5 242 391
- US-A1- 2011 218 488
- US-A1- 2017 079 761

## Description

### BACKGROUND

### Technical Field

This disclosure is generally in the field of medical devices for introduction into the body of a patient, and more particularly in the field of inserter devices and methods for deploying intravesical devices through the urethra and into the urinary bladder.

### Description of Related Prior Art

Intravesical devices may be used to deliver drugs or sense conditions within the bladder. Devices and systems for placement of such devices into a patient's bladder are described, for example, in U.S. Patent No. 8,721,621.

US5242391A discusses how erectile dysfunction, particularly impotence, priapism and Peyronie's disease is treated by the transurethral administration of a therapeutically effective agent. The agents are administered to the urethra by means of a penile insert having a rapidly releasing coating containing the agent on its exterior surface or by means of an inserter carrying an agent containing dose of agent which can be displaced into the urethra.

WO0066213A1 discusses a device for depositing a non-flowable object or a non-flowable medicament in a body cavity that includes an elongate body which includes an elongate barrel with a passage, configured to receive a non-flowable object or medicament, extending through the body. The passage has an outlet at a free end of the barrel, a portion of the passage being curved. The device also includes an ejector or plunger which can be displaced along the passage to push a non-flowable object or medicament received in the passage out of the passage through the outlet thereof.

US2011218488A1 discusses an implantable drug delivery device that includes a drug reservoir portion, a retention frame portion, and a guide wire portion. The drug reservoir portion houses a drug. The retention frame portion houses a retention frame. The guide wire portion defines a hollow bore for receiving a guide wire.

US2017079761A1 discusses an inflated implant, such as an attenuation device, previously implanted in a urinary bladder that can later be removed according to a number of different methods. Preferably, removal is accomplished transurethrally. In one embodiment, removal is accomplished by reducing the inflated implant from an enlarged profile to a reduced profile so that it may be withdrawn transurethrally by a removal system. The removal system can be configured differently depending upon whether reduction from

the enlarged profile to the reduced profile is accomplished by deflation, compression, and/or other ways.

### BRIEF SUMMARY

In one aspect, an inserter assembly is provided for deploying an intravesical device into a urinary bladder of a patient, wherein the inserter assembly includes a placement catheter defining a lumen, the lumen extending from a proximal catheter end to an exit opening; a receiving funnel secured to the placement catheter at the proximal catheter end; and a stylet removably received through the receiving funnel within the lumen of the placement catheter, wherein the stylet comprises (i) a stylet body extending between a proximal stylet end and a distal stylet end, the stylet body being dimensioned to drive the intravesical device through the lumen and the exit opening into the bladder, and (ii) a paddle fixed to the stylet body about the proximal stylet end, wherein the paddle is engageable with the receiving funnel.

In another aspect, an inserter assembly is provided for deploying an intravesical device into a urinary bladder of a patient, wherein the inserter assembly includes a placement catheter defining a lumen, the lumen extending from a proximal catheter end to an exit opening; and a stylet removably received through the lumen of the placement catheter, wherein the stylet comprises a stylet body extending between a proximal stylet end and a distal stylet end, the stylet body being dimensioned to drive the intravesical device through the lumen and the exit opening into the bladder, and a paddle fixed to the stylet

body about the proximal stylet end, wherein the paddle comprises a central body portion surrounding the proximal stylet end, a thumb rest defined at a proximal paddle end, and a pair of wing portions extending laterally from the central body between the thumb rest and a distal paddle end. Disclosed methods are not recited by the wording of the claims (but are considered useful for understanding the invention).

In yet another aspect, a method is provided for deploying an intravesical device in a bladder using an inserter assembly including a placement catheter and a stylet, wherein the method includes (i) introducing the intravesical device in a lumen of the placement catheter through a receiving funnel of the inserter assembly, wherein an exit opening of the deployment body is present in an interior volume of the bladder; (ii) introducing a distal stylet end of the stylet in the lumen of the placement catheter through the receiving funnel to contact the intravesical device within the lumen; and (iii) distally advancing the stylet through the receiving funnel to drive the intravesical device along the lumen toward the exit opening and to release the intravesical device through the exit opening into the bladder.

### BRIEF DESCRIPTION OF THE DRAWINGS

Various embodiments may utilize elements and/or components other than those illustrated in the drawings, and some elements and/or components may not be present in various embodiments. Elements and/or components in the figures are not necessarily drawn to scale. Throughout this disclosure, depending on the context, singular and plural terminology may be used interchangeably.
**FIG. 1A** is a plan view of an embodiment of an inserter assembly including a placement catheter, a receiving funnel, and a stylet.
**FIG. 1B** is a cross-sectional view of the placement catheter shown in **FIG. 1A**, taken along line A-A.
**FIG. 2A** is a front view of a placement catheter of an inserter assembly, according to an embodiment of the present disclosure.
**FIG. 2B** is a side view of the inserter assembly of **FIG. 2A****.**
**FIG. 3A** is a perspective view of a hub portion of an inserter assembly, including a proximal end portion of a placement catheter, a receiving funnel, and a stylet, according to an embodiment of the present disclosure.
**FIG. 3B** is a top end view of the inserter assembly of **FIG. 3A**.
**FIG. 3C** is a plan view of the proximal portion of the inserter assembly of **FIG. 3A**.
**FIG. 4A** is a perspective view of a proximal portion of an inserter assembly, including a proximal end portion of a placement catheter with hub having an asymmetric receiving funnel, and a stylet, according to another embodiment of the present disclosure.
**FIG. 4B** is a plan view of a proximal portion of the inserter assembly of **FIG. 4A** in a first configuration, in which the stylet is partially inserted into the placement catheter.
**FIG. 4C** is a plan view of the proximal portion of the inserter assembly of **FIG. 4A** in a second configuration, in which the stylet is fully inserted into the placement catheter.
**FIG. 4D** is a partial cross-sectional view of the proximal portion of the inserter assembly of **FIG. 4C****,** showing snap fit engagement between the hub/funnel and a fitting on the paddle of the stylet.
**FIGS. 5A** and **5B** are views illustrating manual manipulation of the proximal portion of the inserter assembly shown in **FIGS. 4A** to **4D****.**
**FIGS. 6A** and **6B** are side and top views, respectively, of a proximal portion of an inserter assembly including hub having an asymmetric receiving funnel and a stylet having a paddle with a curved thumb rest at its end, according to an embodiment of the present disclosure.
**FIGS. 7A** and **7B** are side and top views, respectively, of a proximal portion of an inserter assembly including a hub having a symmetric receiving funnel and a stylet having a paddle with a flat end.
**FIG. 8** is a partial side view of an embodiment of an inserter assembly including a placement catheter and a stylet without a fitting, and without a receiving funnel.
**FIG. 9** is a block diagram of an embodiment of a method of deploying an intravesical device within a patient's bladder.

### DETAILED DESCRIPTION

It would be desirable to provide improved inserter devices that provides better, more secure user handling of the inserter device components, that facilitates handling of a lubricant used with the inserter device, that provides better cooperation between the luminal body and the stylet, that provides enhanced operational feedback, including certainty of intravesical device deployment, or a combination thereof.

Improved inserter devices have been developed for placement of intravesical devices into the bladder of a patient. The devices are assemblies of a placement catheter component and a stylet component that cooperate to release an intravesical device from a contained position within a lumen of the placement catheter to a deployed position outside of the placement catheter.

The intravesical device used with the presently disclosed inserter devices may be any medical device suitable for deployment within a patient's bladder. In some embodiments, the intravesical device is a drug delivery device. Examples of such drug delivery devices are described in U.S. Patent No. 10,543,166, U.S. Patent No. 9,283,361, U.S. Patent No. 10,729,823, U.S. Patent No. 10,315,019, U.S. Patent No. 10,737,078, and U.S. Patent No. 11,020,575. In some embodiments, the intravesical device includes diagnostic components, such as cameras and sensors. Examples of such devices are described in U.S. Patent No. 11,065,426 and U.S. Publication No. 2021/0196124.

The terms "distal" or "distally" generally refer to a direction toward the patient, while the terms "proximal" or "proximally" generally refer to a direction away from the patient and toward a clinician. The patient may be a human in need of medical diagnosis, treatment, or prophylaxis.

The present inserter assembly includes a placement catheter, a receiving funnel, and a stylet. The placement catheter defines a lumen extending from a proximal catheter end to an exit opening. The exit opening is at or near a distal end of the placement catheter. The intravesical device may be advanced through the lumen to the exit opening and into the bladder, driven by the stylet.

The stylet is configured to drive one or more intravesical devices through the lumen of the placement catheter into the bladder. For example, an intravesical device may be present or introduced in the lumen, and the stylet may be introduced into the lumen to drive the intravesical device along the lumen. In particular, a distal end of the stylet pushes against a surface, such as an end, of the intravesical device to displace the intravesical device within the lumen toward and through an exit opening in the placement catheter.

The receiving funnel includes a hub that is secured to the placement catheter at the proximal catheter end. The receiving funnel may facilitate introducing the stylet into a proximal end of the catheter, for example, without needing precise alignment between the catheter and the stylet. For example, a tip or end of the stylet may be guided along an interior of the funnel toward a proximal opening and into a lumen of the catheter. Likewise, the receiving funnel also may facilitate introducing the intravesical device into the lumen of the catheter, for example, prior to introduction of the stylet. Further, the receiving funnel beneficially may aid the clinician in introducing a lubricant into the lumen, without requiring use of a syringe to direct the lubricant into the catheter lumen. For example, precisely locating the fine or narrow opening of the lumen may not be required to introduce the lubricant, because the funnel interior and surface would guide the lubricant to the opening.

The stylet is removably received through the receiving funnel within the lumen of the placement catheter. The stylet includes a stylet body extending between a proximal stylet end and a distal stylet end. The stylet body is dimensioned to drive the intravesical device through the lumen and the exit opening into the bladder.

The stylet further advantageously includes a paddle at the proximal stylet end to aid secure handling and manipulation of the stylet, which may particularly be needed when the clinician's gloved fingers may have lubricant on them. The paddle defines two opposed faces extending between paddle ends, for example, faces that can be grasped, pinched, or held. The surface of the paddle may include ridges or texture to facilitate a user's grip of it. The paddle is sometimes referred to as a "hub" of the stylet.

In some embodiments, the paddle includes a fitting that is configured to lockingly engage with the receiving funnel to removably secure the stylet to the placement catheter. The fitting may provide positive (tactile and/or audible) feedback to the clinician when the stylet is fully inserted into the catheter. For example, the lockably engageable fitting may include one or more mechanical features for snap-lock engagement with complementary mechanical features on the receiving funnel. In preferred embodiments, the lockable engagement feature can be manipulated to unlock and disengage the stylet (e.g., the fitting) from the placement catheter (e.g., the receiving funnel).

In some other embodiments, the paddle does not include a locking feature. The placement catheter and stylet may be configured such that during insertion of the stylet, the fitting of the stylet is engageable with the receiving funnel. For example, the fitting may be brought into stopping contact with an interior surface within the receiving funnel, without locking, wherein the stylet subsequently can be freely withdrawn from the placement catheter.

The stylet has a length sufficient to completely drive the intravesical device out through the exit opening when the stylet is fully inserted. Thus, the positive feedback also provides a positive indication that the intravesical device is deployed into the bladder. The engagement of the paddle with the funnel, or the optional lockable engagement of the fitting with the funnel, may also prevent over-insertion of the stylet relative to the catheter, which may prevent unintended introduction of the stylet into the interior of the bladder beyond the end of the catheter, or unintended contact of the stylet and a wall of the bladder. The engagement of the paddle with the funnel may take the form of a distal surface end of the paddle surrounding the stylet coming into interfacing contact with a proximal end surface of the placement catheter, which completes/stops the insertion.

An intravesical device may be placed in a patient's bladder using one of the inserter assemblies described herein. In some embodiments, the method includes introducing the intravesical device in an elongated deployment configuration in the lumen of the placement catheter through the receiving funnel. The exit opening is present in an interior volume of the bladder. The method further includes introducing a distal stylet end in the lumen of the placement catheter through the receiving funnel to contact the intravesical device within the lumen. The method further includes distally advancing the stylet through the receiving funnel to drive the intravesical device along the lumen toward the exit opening and to release the intravesical device through the exit opening into the bladder. The method optionally further includes engaging the paddle of the stylet with the receiving funnel, which may serve to indicate that the distal end of the stylet has reached a position has reached a position at/near the exit opening, to thereby indicate that the intravesical device necessarily has been released into the bladder. In some other embodiments, the paddle need not engage with the funnel for the intravesical device to be released from the placement catheter.

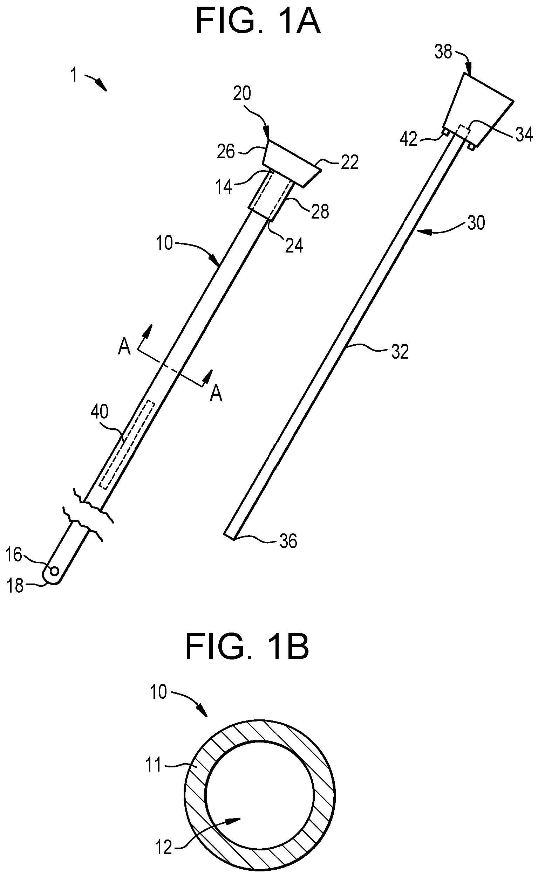

Embodiments of inserter assemblies are shown in **FIGS. 1A** through **8**. Generally, the inserter assemblies include a placement catheter that is designed to pass through a patient's urethra, and a stylet introducible in the placement catheter.

**FIG. 1A** shows an inserter assembly **1** including a placement catheter **10**, a receiving funnel **20,** and a stylet **30.** **FIG. 1B** shows the cross-section of the placement catheter **10** shown in **FIG. 1A****,** taken along line A-A. The inserter assembly **1** may be used to deploy an intravesical device **40.** An intravesical device **40** is shown disposed within a lumen of the placement catheter **10.** The intravesical device **40** may or may not be initially present in the inserter assembly **1.** For example, a clinician may introduce the intravesical device **40** into the placement catheter **10** of the inserter assembly **1** during or shortly before deployment. In some embodiments, the intravesical device **40** may be provided as part of the inserter assembly **1,** for example, within the placement catheter **10,** without needing introduction of the intravesical device **40** into the inserter assembly **1** by the clinician. The cross-sectional shape of the lumen **12** may match the cross-sectional shape of the intravesical device **40.**

In some embodiments, the inserter assembly **1** includes the placement catheter **10,** the receiving funnel **20,** and the stylet **30.** The placement catheter **10** defines a lumen **12** extending from a proximal catheter end **14** to an exit opening **16.** In some embodiments, the exit opening **16** may be adjacent a distal catheter end **18.** The exit opening **16** is in communication with the lumen **12** in a manner that facilitates smooth passage of the intravesical device **40** from the lumen **12** through the exit opening **16.**

In some other embodiments, the exit opening may be at the distal end of the placement catheter and aligned with the lumen.

A wall **11** of the placement catheter **10** generally defines the lumen **12.** The wall **11** is formed from a biocompatible polymeric material, with a thickness providing a column strength suited to prevent buckling of the wall while the placement catheter is being inserted through a urethra. The material preferably is amenable to conventional sterilization processes, such as gamma irradiation or ethylene oxide sterilization. In certain embodiments, the placement catheter **10** is transparent or translucent to visually determine the position of the stylet **30** and/or the intravesical device **40** within the lumen **12.**

The lumen **12** may have a circular, elliptical, or oval cross-sectional profile, or any other suitable profile. The intravesical device **40** may be positioned within the lumen **12,** ready for deployment. Thus, in certain embodiments, the intravesical device **40** may be preloaded within the lumen **12** of the placement catheter **10.** For example, the intravesical device **40** may be preloaded or loaded by the clinician or an apparatus before use into the lumen **12** through the exit opening **16,** or through a proximal opening of the lumen **12** through the receiving funnel **20,** or in an alternative embodiment (not shown) through a slit along the wall **11.**

Generally, the distal catheter end **18** and the distal portion of the placement catheter **10** is configured for traversing the urethra with no or minimal trauma to urethral tissues. The proximal catheter end portion **14** and a proximal portion of the placement catheter **10** remains outside of the body, providing access for a user, such as a medical professional.

The dimensions of the placement catheter **10** may be selected based at least in part on the dimensions of the anatomy that the inserter assembly **1** is designed to navigate. The placement catheter **10** may have any suitable length. In certain embodiments, the placement catheter **10** has a length of at least 10 cm, or of at least 20 cm, or of at least 30 cm, or of at least 40 cm, and less than 60 cm, or less than 80 cm. In certain embodiments, the placement catheter has a length of from 10 cm to 40 cm.

The placement catheter **10** may have any suitable outer diameter. In certain embodiments, the placement catheter has an outer diameter of from 5 Fr (1.67 mm outer diameter) to 18 Fr (6 mm outer diameter). In some other embodiments, the placement catheter may have an outer diameter that is outside the range of 5 to 18 Fr, for example 20, Fr, 22, Fr, 24, Fr, 26 Fr, 28 Fr, or 30 Fr.

One or both of the stylet **30** and the intravesical device **40** may be introduced into the lumen **12** of the placement catheter **10** via or through the receiving funnel **20.** The receiving funnel **20** may have a shape which expands from a narrow end to a wide end. In some embodiments, the receiving funnel **20** may define an aperture adjacent the narrow end that is fluidically coupled to the lumen **12** of the placement catheter. The receiving funnel **20** may have a height that is shallower than one or both of a length or a width of the funnel. In some embodiments, the receiving funnel may have a height having a ratio to a width of from 0.25 to 0.5.

The receiving funnel **20** may be removably securable to the placement catheter **10** by any suitable coupling mechanism, for example, by one or more clips, friction fit, or a pressure sensitive adhesive. In certain embodiments, the receiving funnel **20** is formed of a material that is softer than the material of the placement catheter **10,** such that the receiving funnel can be elastically or plastically pushed, deformed, or placed about the placement catheter **10.** In certain embodiments, the receiving funnel **20** is permanently secured to the placement catheter **10,** for example, by molding, overmolding, or permanent adhesive, or any suitable mechanism that secures the receiving funnel **20** the placement catheter **10** in a manner that cannot be removed nondestructively.

In certain embodiments, the receiving funnel **20** is overmolded about the placement catheter **10** at the proximal catheter end **14.** In certain embodiments, the receiving funnel **20** is integral with the placement catheter **10** at the proximal catheter end **14.** As used herein, the terms "integral with" and "integrally formed" refer to the funnel and placement catheter being part of the same monolithic structure/material, e.g., co-molded. The receiving funnel **20** may be secured to the placement catheter **10** at, adjacent, or about the proximal catheter end **14.** In certain embodiments, the receiving funnel **20** is integrally formed with the placement catheter **10.** For example, a rear or proximal portion of the placement catheter may be formed as the receiving funnel **20.**

The receiving funnel **20** extends between a proximal funnel end **22** and a distal funnel end **24.** The receiving funnel **20** may consist of a single or integral unit extending between the proximal funnel end **22** and a distal funnel end **24.** In certain embodiments, two or more units may be separately formed and secured to form the receiving funnel **20.** For example, the receiving funnel **20** may include a funnel portion **26** and a hub **28** that can be integrally formed as a single unit, or may be separately formed and secured to each other. The hub **28** may be dimensioned to be secured to or about the proximal catheter end **14** of the placement catheter **10.** The funnel portion **26** may be dimensioned to widen in a proximal direction away from the proximal catheter end **14** to facilitate receiving the stylet **30,** the intravesical device **40,** or a fluid, such as a lubricant, within the lumen **12** of the placement catheter **10.**

In the embodiment shown in **FIG. 1A****,** the narrowest width of the funnel portion **26** is wider than the width of the hub **28.** In certain embodiments, the width of the hub **28** may increase to smoothly transition into a width of the funnel portion **26.** Thus, the exterior surface of the funnel portion **26** may smoothly extend into the exterior surface of the hub **28.**

The receiving funnel **20** may have any suitable profile, for example, a profile defined by a curve, a contour, a polygon, a linear or piecewise linear path, or combinations thereof. In certain embodiments, the receiving funnel **20** has a circular or oval profile. In certain embodiments, the receiving funnel **20** is flattened, such that the receiving funnel **20** has a shorter minor axis and a wider major axis in a direction transverse to the receiving funnel **20.** In certain embodiments, the receiving funnel is dimensioned to receive at least 0.1 mL, or at least 0.5 mL, or at least 1 mL of a liquid or gel, e.g., a lubricant.

The funnel portion **26** defines a funnel volume fluidically coupled to the lumen **12** of the placement catheter **10.** Thus, the receiving funnel **20** may be used to guide components or fluids into the lumen **12** without requiring an exact determination of the location or alignment of a proximal opening of the lumen **12.** The proximal catheter end **14,** as shown in **FIG. 1A****,** may extend to and align with an interface of the funnel portion **26** and the hub **28**. Thus, a proximal opening of the lumen **12** may align with that interface. In certain embodiments, the proximal catheter end **14** may not extend to that interface, and may instead, terminate at a position along the hub **28.**

In certain embodiments, the receiving funnel **20** is soft and elastically deformable, for example, in response to a force exerted by the clinician. In other embodiments, the receiving funnel **20** is rigid, and substantially retains its shape in response to a force exerted by the clinician.

A proximal portion of the stylet **30** adjacent the proximal stylet end **34** remains outside of the patient's body and is configured for receiving a driving force from a clinician or an apparatus. A distal portion adjacent the distal stylet end **36** is configured for driving the intravesical device **40** from the exit opening **16.**

The stylet **30** is removably received through the receiving funnel **20** within the lumen **12** of the placement catheter **10.** The stylet **30** includes a stylet body **32** extending between a proximal stylet end **34** and a distal stylet end **36.** The stylet body **32** is dimensioned to drive the intravesical device **40** through the lumen **12** and the exit opening **16** into the patient's bladder.

The overall shape and configuration of the stylet **30** permits applying the driving force to the intravesical device **40** without substantial friction between the placement catheter **10** and the stylet **30** and without the stylet **30** causing trauma to the placement catheter **10** or to the intravesical device **40.**

The dimensions of the stylet **30** may be selected based at least in part on the dimensions of the placement catheter **10** lumen and the size of the intravesical device. The stylet **30** may be slightly longer than the placement catheter **10.** Once inserted into the deployment instrument, the stylet proximal end **34** remains outside of the proximal catheter end **14** of the placement catheter **10,** and the distal stylet end **36** reaches to the distal catheter end **18** of the placement catheter **10.** Such a configuration permits inserting the stylet **30** along the full length of the placement catheter **10** to drive the intravesical device **40** from the exit opening **16.**

An outer diameter of the stylet **30** is smaller than an inner diameter of the placement catheter **10,** so that the stylet **30** can be inserted into the deployment instrument without having to overcome the force of friction along the length of the entire placement catheter **10.** In some embodiments, the stylet body has an outer diameter that is about 1 mm less than the inner diameter of the lumen of the placement catheter.

The stylet **30** is formed from a suitable material that has a column strength suited to resist buckling during insertion into the placement catheter lumen and driving of the intravesical device. The material may be a biocompatible polymer, such as silicone, polyethylene, or polyurethane and preferably is amenable to conventional sterilization procedures. The stylet **30** can be formed, for example, by extrusion or molding processes known in the art.

In the illustrated embodiment, the stylet **30** has a substantially cylindrical outer surface, although other configurations are possible.

In certain embodiments, the stylet body **32** has a solid cross-section along an entirety of the stylet body **32**. In other embodiments, the stylet body **32** may be hollow. For example, the stylet body **32** may define a lumen extending from the proximal stylet end **34** to the distal stylet end **36**. In some such embodiments, the lumen of the stylet body **32** may be used to permit passage of fluids into or out of the placement catheter lumen and/or the bladder. In some embodiments, the lumen may extend through the paddle.

The stylet **30** further includes a paddle **38** at, adjacent, or about the proximal stylet end **34.** The paddle **38** is engageable with the receiving funnel **20,** for example, by contact between surfaces of the paddle **38** and the receiving funnel **20.** Optionally, the paddle **38** is lockably engageable with the receiving funnel **20** to removably secure the stylet **30** to the receiving funnel **30** and ultimately to the placement catheter **10.**

The paddle **38** optionally includes at least one fitting **42** for promoting lockable engagement of the paddle **38** to the funnel **20.** The fitting **42** may include at least one clip, for example, one, two or more clips. In certain embodiments, the fitting **42** includes at least one clip removably securable to the receiving funnel **30.** For example, the at least one clip may be securable to a surface or a feature of the receiving funnel **30,** for example, a lip, a rim, a contour, a protrusion, a channel, a ridge, an opening, or any suitable feature complementary to the at least one clip (or generally to the fitting **42**). In certain embodiments, the fitting **42** is removably securable to or at an interior surface of the receiving funnel **20,** for example, adjacent the proximal catheter end **14.** The fitting **42** may exert a biasing or securing force such that once stylet **30** is fully engaged and secured to the receiving funnel **20** (and thus to the placement catheter **10**), the stylet **30** will remain secured to the receiving funnel **20** until at least a minimum threshold removing force is exerted on the stylet **30** in a proximal direction away from the placement catheter **10.**

In certain embodiments, the fitting **42** generates a tactile or audible feedback upon engagement. In certain embodiments, the stylet **30** itself is configured to generate audible or tactile feedback during engagement with the receiving funnel **20,** for example, by snapping or tapping against the receiving funnel **20.** Thus, the fitting **42** may facilitate a complete engagement and securement of the stylet **30** to the placement catheter **10,** until intentionally released by the clinician.

Instead of, or in addition to fitting **42** including a clip, the inserter assembly **1** may include other mechanisms for securing the stylet **30** to the placement catheter **10.** For example, one of the fitting **42** or the receiving funnel **20** may define a circumferential ridge, and the other of the fitting **42** or the receiving funnel **20** may define a circumferential channel dimensioned to matingly engage the circumferential ridge. The mating ridge and channel may be suitably elastic and dimensioned to provide a "snap-fit" locking engagement.

In some other embodiments, the fitting **42** and the receiving funnel **20** may be securable by a luer lock or a twist-lock.

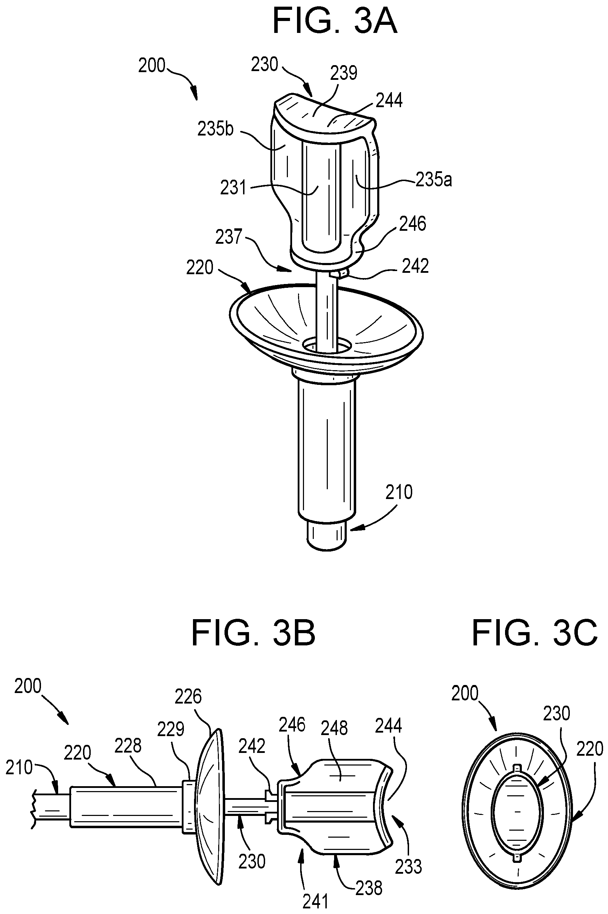

While in the embodiment of **FIG. 1A****,** the fitting **42** is associated with the stylet **30,** in other embodiments, a fitting may be provided on one or both of the stylet **30** (for example, at the paddle **38**) and the receiving funnel **20,** securable to the other of the stylet **30** (or the paddle **38**) and the receiving funnel **20.** In certain embodiments, the fitting **42** includes two clips distally extending from a distal paddle end and securable to an interior funnel lip. Further description of the fitting and funnel engagement are described below with reference to FIGS. 3A to 7B.

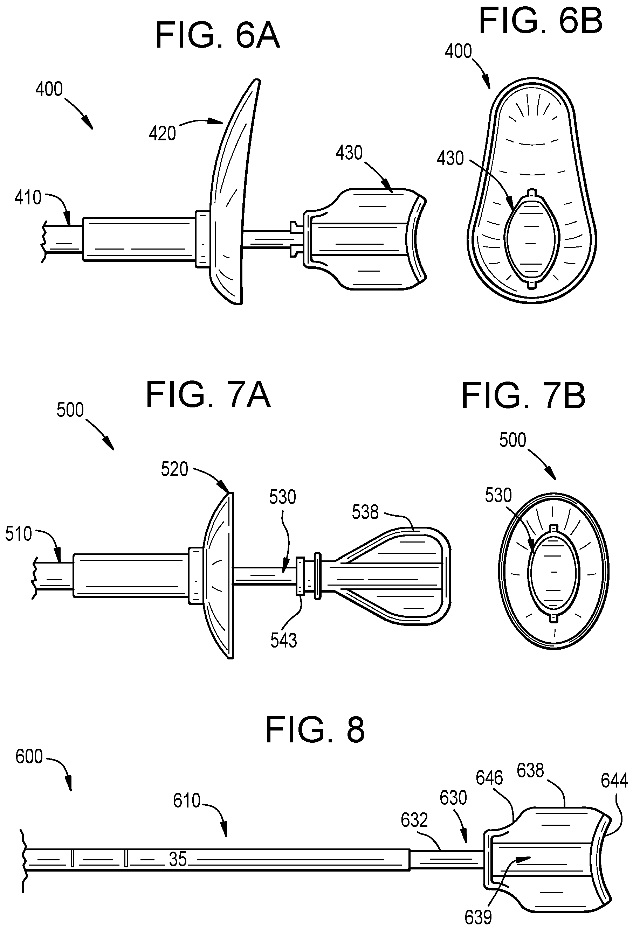

In certain other embodiments, an inserter assembly may not include a fitting, as described below with reference to FIG. 8.

The paddle **38** may be secured to the stylet body **32,** for example, by a friction or interference fit, molding, overmolding, or permanent adhesive, or any suitable mechanism that secures the paddle **38** to the stylet body **32,** for example, in a manner that cannot be removed by a clinician. The proximal stylet end **34** may extend to and align with an end of the paddle **38.** Thus, the proximal ends of the stylet body **32** and the paddle **38** may be flush or aligned. In certain embodiments, the proximal stylet end **34** may not extend in such a manner and instead may terminate at a position within an interior of the paddle **38,** as shown in **FIG. 1A****.** In certain embodiments, the paddle **38** is overmolded about the stylet body **32** at the proximal stylet end **34.** In certain embodiments, the paddle **38** may be coupled to the stylet body by an adapter. In certain embodiments, the paddle **38** is integral with the stylet body **32.** For example, the stylet body **32** and the paddle **38** may be molded together, or as a single unit or piece.

The paddle **38** is dimensioned to engage with the receiving funnel **20.** For example, the paddle **38** may be wider or larger than the receiving funnel **20** in at least one dimension, such that the paddle **38** may be secured at or about an exterior surface, rim, or lip of the receiving funnel **20.** In certain embodiments, the paddle **38** is narrower or smaller than the receiving funnel **20** in at least one dimension, such that the paddle **38** is at least partially received within and engaged against an interior surface of the funnel portion **26.** In certain such embodiments, a portion of the paddle **38** may extend proximally outward the funnel portion **26,** such that a clinician may grasp, pinch, or grab the extending portion and exert a pulling force on the paddle **38** to disengage or remove the paddle **38** (and thus the stylet **30**) from the receiving funnel **20.** In certain embodiments, no portion of the paddle **38** extends out from the receiving funnel **20,** so that the clinician cannot easily manually remove or disengage the paddle **38** (and thus the stylet **30**), for example, without using a tool such as a forceps. Such a configuration may be used to prevent or avoid unintentional disengagement or removal of the stylet **30** from the placement catheter **10.**

In certain embodiments, the placement catheter **10** defines a rounded atraumatic tip at the distal catheter end **18,** for example, as shown in **FIG. 1A****.** In other embodiments, the distal end of the placement catheter **10** may have a relatively flat or tapered tip so long as suitably configured to permit insertion into/through the urethra.

One or more of the placement catheter **10,** the paddle **38,** the receiving funnel **20,** and the stylet **1** may be formed of the same or different polymeric material. In certain embodiments, the inserter assembly **1** includes a radiomarker. For example, the placement catheter **10** or the stylet body **32** may include a radio-opaque material or marker.

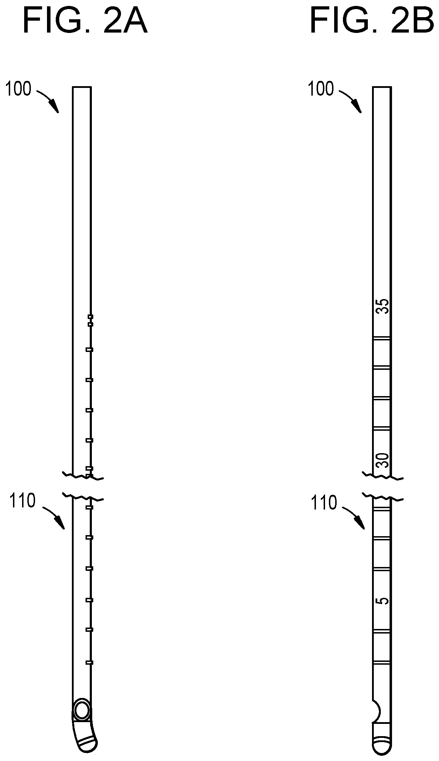

The inserter assembly **1** may be used to drive and deploy an intravesical device **40.** Further embodiments of inserter assemblies or components thereof are described with reference to **FIGS. 2A** to **8**, with similarly numbered reference numerals designating like elements. It will be understood that the inserter assembly **1** may include components modified according to such and other embodiments.

**FIGS**. **2A-2B** illustrate one embodiment of a placement catheter **110** of an inserter assembly **100.** The placement catheter **110** has a rounded atraumatic tip including a coudé bend. When a coudé bend is present, the coudé bend may be aligned at a predetermined angle relative (for example, aligned with, or normal to) a major axis of the retrieval funnel such that an orientation of the retrieval funnel is indicative of an orientation of the coudé bend. In certain embodiments, the placement catheter **110** may include depth markings indicative of insertion depth and exit opening orientation, as shown in **FIG. 2B****.**

One preferred embodiment of an inserter assembly **200** is shown in **FIGS. 3A-3C****.** The placement catheter **210** may be generally similar to the placement catheter **10** described with reference to **FIGS. 1A-1B** or the placement catheter **110** described with reference to **FIGS. 2A- 2B****.** The funnel portion **226** of the receiving funnel **220** has an oval profile, as seen in **FIGS. 3B** and **3C****.** A rim **229** may be present at an external interface of the funnel portion **226** and the hub **228.**

Stylet **230** includes a paddle **238** including a fitting **242.** The fitting **242** includes two clips, extending distally from the fitting **238.** The two clips of the fitting **242** are received within and removably securable to an interior of the receiving funnel **220.**

The paddle **238** has a central body portion **231** surrounding the proximal stylet end, a thumb rest **244** defined at a proximal paddle end **233,** and a pair of wing portions **235a**, **235b** extending laterally from the central body portion **231** between the thumb rest **244** and a distal paddle end **237.** The thumb rest **244** has a concave surface **239** facing away from the central body portion **231** and an oval shaped periphery, the longer axis of which extends the same direction as the wing portions extend from the central body portion. Each of the wing portions **235a**, **235b** includes a taper **241** leading to the distal paddle end **237.** The paddle **238** also includes a curved finger rest **246** proximate to each taper **241.** Surface areas of the wing portions **235a**, **235b** optionally include a grip surface **248** extending between the proximal paddle end and the distal paddle end. For example, the grip surface may include a non-slip coating material known in the art or a texturized area (e.g., a plurality of ridges, grooves, raised bumps, or other surface roughening features). One or more of the thumb rest **244,** curved finger rest **246,** and the grip surface **248** may facilitate holding, grasping, grabbing, pulling, and pushing of the stylet **230** relative to the placement catheter **210** and the receiving funnel **220.**

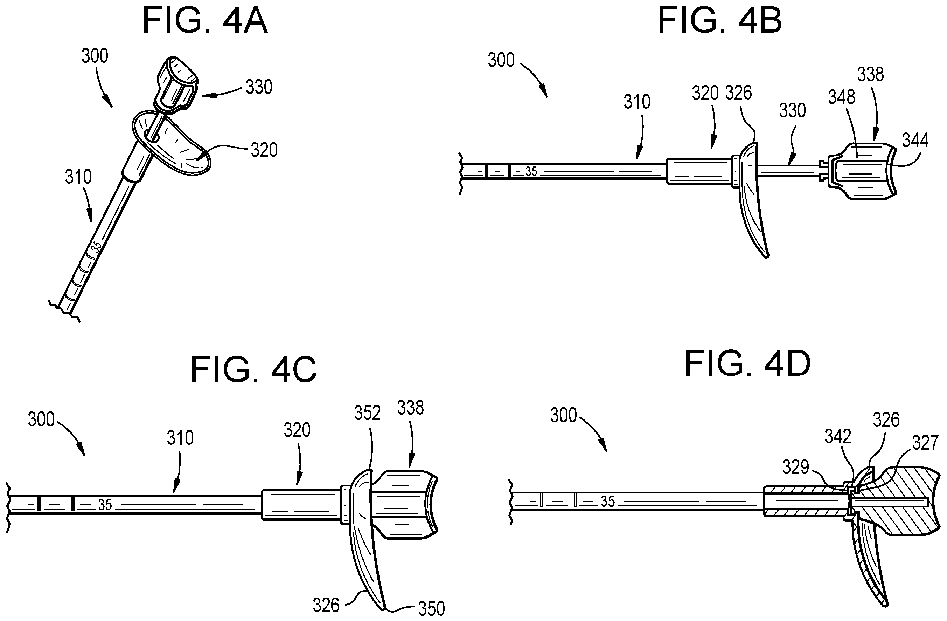

Another preferred embodiment of an inserter assembly **300** is shown in **FIGS. 4A-****4D.** The inserter assembly **300** is substantially similar to the inserter assembly **200** described with reference to **FIGS. 3A** to **3C****,** but with the receiving funnel **320** extending asymmetrically in a direction transverse to the stylet body **330.**

As shown in **FIG. 4A** the inserter assembly **300** includes a placement catheter **310,** an asymmetric receiving funnel **320,** and a stylet **330.** In a first illustrated configuration, the stylet **330** is partially inserted into placement catheter **310,** as shown in **FIGS. 4A-4B****.** In a second illustrated configuration, the stylet **330** is fully inserted into the lumen of the placement catheter **310,** as shown in **FIGS. 4C-4D****.**

The placement catheter **310** includes depth markings, similar to the placement catheter **110** shown in **FIGS. 2A-2B****.** A first funnel edge **350** of the funnel portion **326** of the receiving funnel **320** is proximally offset and extends further proximally relative a second funnel edge **352,** as shown in **FIG. 4C****.** The paddle **338** includes the thumb rest **344** and the gripping surface **348,** as shown in **FIG. 4B****.**

The paddle **338** includes a fitting **342** including two clips securable to the inner rim **327** in an interior of the funnel portion **326,** and received within a channel **329** adjacent the inner rim **327,** as shown in **FIG. 4D****.**

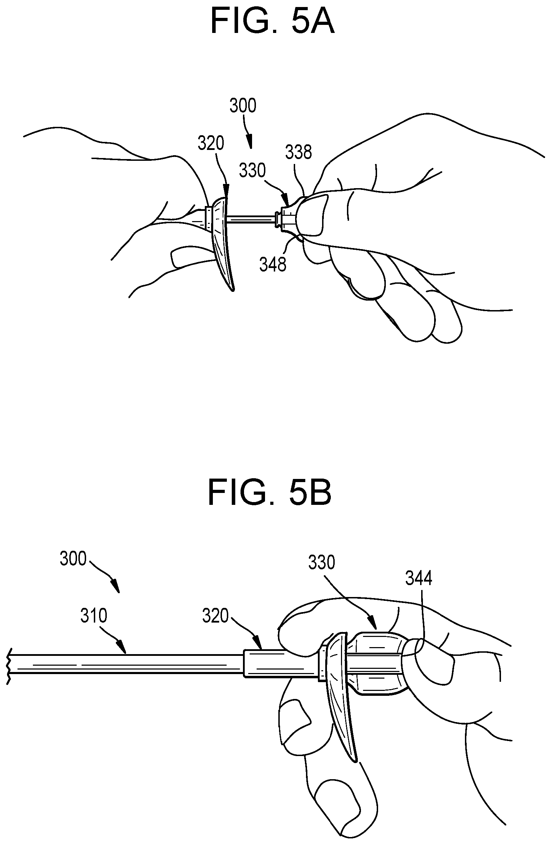

The asymmetric configuration of the receiving funnel **320** of the inserter assembly **300** may facilitate ergonomical manual operation of the inserter assembly **300,** as shown in **FIGS. 5A-5B. FIG. 5A** is a view illustrating a use of the inserter assembly **300** of **FIG. 4A** with the stylet **330** partially inserted through the placement catheter **310.** **FIG. 5B** is a view illustrating a use of the inserter assembly **300** of **FIG. 4A** with the stylet **330** fully inserted. As shown in **FIG. 5A****,** the thumb of one hand of a user can rest against the asymmetric portion of the receiving funnel **320,** with the thumb and fingers of the user's other hand gripping the grip surface **348** of the paddle **338.** This configuration can be used to either advance or retract the stylet **330** distally or proximally relative to the funnel **320.** To completely insert the stylet **330,** the thumb of one hand can exert a final push on the curbed thumb rest **344** of the stylet **330,** with fingers resting against the receiving funnel **320,** as shown in **FIG. 5B****.**

Another preferred embodiment of an inserter assembly **400** is shown in **FIGS. 6A-****6B.** The inserter assembly **400** includes a placement catheter **410,** an asymmetric receiving funnel **420,** and a stylet **430.** The inserter assembly **400** is substantially similar to the inserter assembly **300** described with reference to **FIGS. 4A-4D** but with the receiving funnel **420** having a pear-shaped asymmetric profile in a direction transverse to the stylet body **430.** The shape of the receiving funnel **420** may facilitating holding of the asymmetric funnel **420,** for example, between fingers or between fingers and thumb.

Another preferred embodiment of an inserter assembly **500** is shown in **FIGS. 7A-****7B.** The inserter assembly **500** including a placement catheter **510,** a symmetric receiving funnel **520,** and a stylet **530.** The inserter assembly **500** is substantially similar to the inserter assembly **200** described with reference to **FIGS. 3A-3C** but with the paddle **538** of the inserter assembly **500** having a different shape than the paddle **238** of the inserter assembly **200.** Paddle **538** has a tapered shape similar to a guitar pick, and also includes a twist-lock **543** (e.g., Luer lock) securable within an interior of receiving funnel **520.**

**FIG. 8** illustrates an alternative embodiment of an inserter assembly **600** that includes a placement catheter **610** and a stylet **630** having neither a fitting nor a receiving funnel. Apart from the absence of the fitting or the receiving funnel, the inserter assembly **600** is substantially similar to the inserter assembly **200** described with reference to **FIGS. 3A** to **3C****.** The stylet **630** includes a stylet body **632** extending between a proximal stylet end and a distal stylet end. The stylet **630** includes a paddle **638** fixed to the stylet body **632** about the proximal stylet end. The paddle **638** defines a curved thumb rest **644** at a proximal paddle end, and a curved finger rest **646** at a distal paddle end. The curved thumb rest **644** may extend along a direction transverse to the stylet body **632,** for example, perpendicular to the stylet body **632**, or within ±10° of a direction perpendicular to the stylet body **632.** In embodiments, the curved finger rest **646** is a first curved finger rest, and the paddle **638** further defines a second curved finger rest at the distal paddle end, as shown in **FIG. 8****.**

The paddle **638** is fixed to the stylet body **632.** For example, the paddle **638** may be overmolded about the stylet body **632** at the proximal stylet end. In such embodiments, the paddle **638** has a central body portion **639** defining a paddle lumen in which a proximal end portion of the stylet body **632** extends. In an alternative embodiments, the paddle **638** is integrally formed with the stylet body **632,** such as in an injection molding process.

In some embodiments, the inserter assembly **600** may not include a receiving funnel, for example as shown in **FIG. 8****.** In such embodiments, the paddle **638** may engage with the placement catheter **610.** For example, a distal portion of the paddle **638** may contact or engage with a proximal portion of the placement catheter **610.** Thus, a clinician may stop driving or pushing the stylet body **632** upon receiving audio or tactile feedback generated by engagement of the paddle **638** with the placement catheter **610.** In other embodiments, the inserter assembly **600** may include a receiving funnel according to an embodiment of the disclosure, and the paddle **638** may engage with the receiving funnel.

While embodiments of insertion assemblies including a receiving funnel, a paddle, and a locking feature or fitting have been described, in other embodiments, the insertion assemblies may include one or two of a receiving funnel, a paddle, and a locking feature or fitting. In some embodiments, one or both of a receiving funnel and the fitting may be absent from inserter assemblies. In embodiments in which the receiving funnel is absent, the paddle may engage with or rest against a proximal end of the placement catheter after advancing the stylet through the placement catheter.

Various insertion assemblies according to the present disclosure may be provided as respective kits. For example, the kit may include a package that holds a placement catheter and a stylet. The kit may include a separate hub with integral receiving funnel, securable to the placement catheter prior to use, or the receiving funnel may be bonded or secured to the placement catheter. The kit may include an intravesical device to be introduced within the placement catheter, or may include the intravesical device preloaded within the placement catheter.

In certain embodiments, the deployment assemblies may be housed in at least one package, or kit, that stores one or more of the placement catheter, the receiving funnel, the stylet, and the intravesical device. The package protects the packaged components before the insertion procedure. For example, the components may be sterilized together, and transported together, in the package. One or both of the stylet or the receiving funnel can be either packaged with the placement catheter, packaged separately, or omitted completely. Regardless of which components are packaged together, the package is sterilized, such as using gamma irradiation or ethylene oxide sterilization.

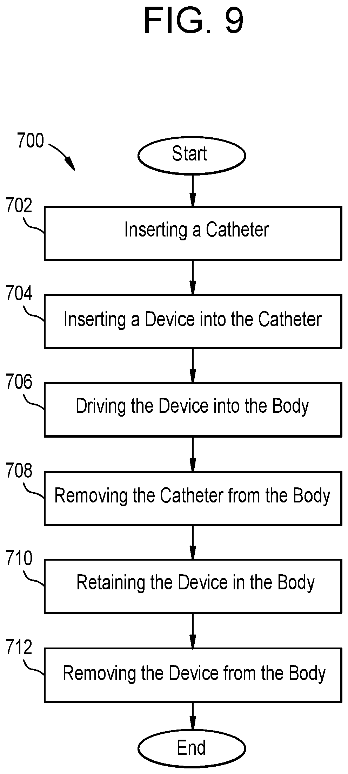

**FIG. 9** is a block diagram of an embodiment of a method of inserting an intravesical device in the bladder. The method of **FIG. 9** may be practiced using any insertion assembly according to the present disclosure, for example, an insertion assembly including a placement catheter, a receiving funnel, and a stylet.

In block **702,** a placement catheter is inserted into the body. Inserting the placement catheter generally includes inserting the placement catheter into the urethra and driving the placement catheter forward until a distal end is positioned in the bladder, while a proximal end remains outside of the body. For example, the distal stylet end may be introduced in the lumen of the placement catheter through the receiving funnel to contact the intravesical device within the lumen.

In some embodiments, the placement catheter is inserted into the body in block **702** without the use of any additional devices. In some other embodiments, the placement catheter is inserted into the body in block **702** in association with a cystoscope, which permits visualizing the insertion procedure.

In some embodiments, inserting the placement catheter into the body in block **702** further includes verifying a distal end of the placement catheter has become positioned in the bladder. For example, the location of the distal end can be verified by communicating urine through the placement catheter from the distal end positioned in the bladder and/or by using the depth indicator markings (e.g., numbers) on the placement catheter. Alternatively, the location of the distal end may be verified by visualizing the distal end of the placement catheter with a cystoscope, an ultrasound or x-ray.

In embodiments in which the placement catheter includes a coudé bend, or tip, the clinician may rotate the placement catheter, and in turn the coudé bend, to control the orientation at which the intravesical device exits the placement catheter. For example, the coudé bend may orient the exit opening towards a direction that can be controlled by rotating the placement catheter. Thus, in some embodiments, the clinician to may orient, steer, or direct the intravesical device away from any anatomical features in the bladder that could impede release of the intravesical device from placement catheter.

In block **704,** the intravesical device is inserted into the placement catheter. In some cases, inserting the intravesical device into the placement catheter can include elastically deforming the intravesical device from a non-linear bladder retention shape (e.g., a coil shape or an S-shape) to a substantially linear deployment shape. For example, block **704** may include introducing the intravesical device in an elongated deployment configuration in the lumen of the placement catheter through the receiving funnel, wherein the exit opening is present in an interior volume of the bladder.

Inserting the intravesical device into the placement catheter may include inserting the intravesical device into the distal catheter end, into the proximal catheter end, or into an opening or slit along the wall of the placement catheter. In some cases, inserting the intravesical device into the placement catheter comprises pre-loading the intravesical device into the placement catheter before the placement catheter is inserted into the body. In such cases, the order of blocks **702** and **704** is reversed. The intravesical device is typically pre-loaded if the device is placed in the placement catheter through the distal end or slit, although the intravesical device can be pre-loaded in any case for convenience. The intravesical device may be lubricated as, or before, the intravesical device is inserted into the placement catheter, to reduce friction associated with driving the intravesical device through the placement catheter in block **706.**

In block **706,** the intravesical device is driven into the bladder. In embodiments in which the intravesical device is inserted into the placement catheter through the receiving funnel into the proximal catheter end in block **704,** driving the intravesical device into the bladder includes driving the intravesical device from the proximal catheter end, along the lumen, and through the distal catheter end until the intravesical device exits the placement catheter into the bladder. In embodiments in which the intravesical device is inserted into the distal catheter end in block **704,** driving the intravesical device into the bladder includes driving the intravesical device through the distal catheter end until the intravesical device exits the placement catheter into the bladder, as the intravesical device was previously driven through the urethra upon insertion of the placement catheter in block **702.**

Driving the intravesical device into the bladder in block **706** includes driving the intravesical device with a stylet. The stylet may be an embodiment of the stylet described above. In such cases, driving the intravesical device into the bladder in block **706** includes inserting the distal stylet end into the proximal catheter end portion through the receiving funnel and advancing the stylet through the placement catheter until the distal stylet end reaches the distal catheter end. Driving the intravesical device into the bladder in block **706** includes contacting the intravesical device with the stylet, advancing the intravesical device through the placement catheter using the stylet, and driving the intravesical device from the placement catheter with the stylet. For example, the stylet may be distally advanced through the receiving funnel to drive the intravesical device along the lumen toward the exit opening, and the fitting of the stylet or a portion of the paddle may optionally be engaged with the receiving funnel as an indication of complete stylet insertion and release of the intravesical device through the exit opening into the bladder.

In some embodiments in which the method is used to place an intravesical device that is configurable between a deployment shape (e.g., a linear shape) and a retention shape (e.g., a non-linear shape), the act of driving the intravesical device from the placement catheter in block **706** removes the force of the placement catheter wall from the intravesical device. This unloading may permit the intravesical device to elastically return to its retention shape for retaining the intravesical device within the bladder during a medically indicated (e.g., treatment or diagnostic) period. In other words, the driving may remove a restraint or confinement imposed on the intravesical device by the placement catheter wall.

In some embodiments, snap lock engagement of the fitting to the receiving funnel provides tactile and/or audible feedback that the stylet is fully inserted, which thereby ensures that the intravesical device necessarily has been deployed. In some embodiments, another portion of the paddle besides the fitting may contact the funnel, providing tactile and/or audible feedback of stylet insertion. For example, the In some embodiments, driving the intravesical device into the bladder in block **706** may further include observing the intravesical device in the bladder to ensure the device was properly deployed. For example, the device may be observed using a cystoscope, ultrasound, or x-ray.

In some embodiments, driving the intravesical device into the bladder in block **706** further includes introducing a volume of fluid into the bladder before the device is driven into the bladder. The fluid may be, for example, water or saline. The fluid may serve as a cushion in the bladder, reducing the likelihood of the intravesical device contacting the posterior of the bladder upon deployment, which may reduce bladder discomfort.

In block **708,** the placement catheter and stylet are removed from the body. The stylet can be removed be either before or simultaneously with the placement catheter. For example, the paddle may be released from the receiving funnel, and the stylet proximally withdrawn through the lumen and from the catheter. In other embodiments, the paddle may remain engaged with the receiving funnel and the stylet within the lumen during the withdrawing of the catheter from the bladder. Thereafter, in block **710,** the intravesical device remains deployed within the bladder for a period as medically indicated. In embodiments in which the intravesical device is a drug delivery device, the device is retained in the body in block **710** to release a drug into the body, such as to release drug from the device into the bladder and to the urothelial tissues and other local or regional tissues.

In some embodiments, the method includes removing the intravesical device from the body in block **712.** Removing the intravesical device from the body may include inserting a removal instrument into the body, locating the deployed intravesical device in the body, grasping or otherwise securing the intravesical device, and pulling the intravesical device from the body using the removal instrument. The removal instrument may include use of a cystoscope or catheter that is inserted through the urethra until its distal end reaches the bladder, and forceps or other means may be used to facilitate grasping or securing the intravesical device.

## Claims

1. An inserter assembly (1) for deploying an intravesical device (40) into a urinary bladder of a patient, the inserter assembly (1) comprising:
a placement catheter (10) defining a lumen (12), the lumen (12) extending from a proximal catheter end (14) to an exit opening (16);
a receiving funnel (20) secured to the placement catheter (10) at the proximal catheter end; and
a stylet (30) removably received through the receiving funnel (20) within the lumen (12) of the placement catheter (10), wherein the stylet (30) comprises (i) a stylet body (32) extending between a proximal stylet end (34) and a distal stylet end (36), the stylet body (32) being dimensioned to drive the intravesical device (40) through the lumen (12) and the exit opening (16) into the bladder, and (ii) a paddle (38) fixed to the stylet body (32) about the proximal stylet end (34), wherein the paddle (38) is engageable with the receiving funnel (20).

2. The inserter assembly (1) of claim 1, wherein the paddle (38) is overmolded about the stylet body (33) at the proximal stylet end (34), or is integral with the stylet body (34).

3. The inserter assembly (1) of claim 1 or 2, wherein the paddle (38) defines a curved thumb rest (244) at a proximal end (34), and optionally further defines a curved finger rest (246) opposing the curved thumb rest (244).

4. The inserter assembly (1) of any one of claims 1 to 3, wherein the paddle (38) comprises a grip surface (248).

5. The inserter assembly (1) of any one of claims 1 to 4, wherein the paddle (38) is dimensioned to be received within the receiving funnel (20).

6. The inserter assembly (1) of any one of claims 1 to 5, wherein the paddle (38) comprises a fitting (42) being lockably engageable with the receiving funnel (20) to removably secure the stylet (30) to the placement catheter (10), optionally wherein the fitting (42) is engageable with an interior surface of the receiving funnel (20).

7. The inserter assembly (1) of claim 6, wherein:
one or both of the fitting (42) and the receiving funnel (20) comprises at least one clip securable to the other of the fitting (42) and the receiving funnel (20); and/or
the fitting (42) defines a circumferential ridge, and wherein the receiving funnel (20) defines a circumferential channel dimensioned to matingly engage the circumferential ridge.

8. The inserter assembly (1) of claims 6 or 7, wherein the fitting (42) is configured to generate audible and/or tactile feedback during engagement with the receiving funnel (20).

9. The inserter assembly (1) of any one of claims 1 to 8, wherein:
the receiving funnel (20) has one or both of an oval profile and an oval perimeter; and/or
the receiving funnel (20) extends asymmetrically in a direction transverse to the stylet body (32).

10. The inserter assembly (1) of any one of claims 1 to 9, wherein the receiving funnel (20) defines a funnel lumen configured to transfer lubricant from the receiving funnel (20) to the lumen (12) of the placement catheter (10), optionally wherein the receiving funnel (20) is dimensioned to receive at least 1 mL of the lubricant.

11. The inserter assembly (1) of any one of claims 1 to 10, wherein the placement catheter (10) is transparent or translucent to visually determine the position of the intravesical device (40) or the stylet (30) within the lumen (12), and/or wherein the placement catheter (10) comprises depth markings indicative of insertion depth and exit opening (16) orientation.

12. The inserter assembly (1) of any one of claims 1 to 11, wherein a distal catheter end (18) of the placement catheter (10) comprises (i) a rounded atraumatic tip, and/or (ii) a coudé bend, which optionally is aligned with a major axis of the retrieval funnel such that an orientation of the retrieval funnel is indicative of an orientation of the coudé bend.

13. The inserter assembly (1) of any one of claims 1 to 12, wherein the exit opening (16) is at the distal end (18) of the placement catheter (10) and is aligned with the lumen (12).

14. The inserter assembly (1) of any one of claims 1 to 13, wherein the stylet body (32) defines a lumen extending from the proximal stylet end (34) to the distal stylet end (36).

15. The inserter assembly (1) of any one of claims 1 to 14, wherein the paddle (238) comprises:
a central body portion (231) surrounding the proximal stylet end (34);
a thumb rest (244) defined at a proximal paddle end (233); and
a pair of wing portions (235a, 235b) extending laterally from the central body portion (231) between the thumb rest (244) and a distal paddle end (237),
optionally wherein the thumb rest (244) has a concave surface facing away from the central body (231), an oval shaped periphery, or both a concave surface facing away from the central body (321) and an oval shaped periphery.

## Patentansprüche

1. Einführungsanordnung (1) zum Einsetzen einer intravesikalen Vorrichtung (40) in eine Harnblase eines Patienten, die Einführungsanordnung (1) umfassend:
einen Platzierungskatheter (10), der ein Lumen (12) definiert, wobei sich das Lumen (12) von einem proximalen Katheterende (14) zu einer Austrittsöffnung (16) erstreckt;
einen Aufnahmetrichter (20), der an dem Platzierungskatheter (10) an dem proximalen Katheterende befestigt ist; und
ein Stilett (30), das durch den Aufnahmetrichter (20) innerhalb des Lumens (12) des Platzierungskatheters (10) entfernbar aufgenommen ist, wobei das Stilett (30) (i) einen Stilettkörper (32), der sich zwischen einem proximalen Stilettende (34) und einem distalen Stilettende (36) erstreckt, wobei der Stilettkörper (32) bemessen ist, um die intravesikale Vorrichtung (40) durch das Lumen (12) und die Austrittsöffnung (16) in die Blase zu treiben, und (ii) ein Paddel (38) umfasst, das an dem Stilettkörper (32) um das proximale Stilettende (34) fixiert ist, wobei das Paddel (38) mit dem Aufnahmetrichter (20) in Eingriff bringbar ist.

2. Einführungsanordnung (1) nach Anspruch 1, wobei das Paddel (38) um den Stilettkörper (33) an dem proximalen Stilettende (34) umspritzt ist oder mit dem Stilettkörper (34) einstückig ist.

3. Einführungsanordnung (1) nach Anspruch 1 oder 2, wobei das Paddel (38) eine gekrümmte Daumenauflage (244) an einem proximalen Ende (34) definiert und optional ferner eine gekrümmte Fingerauflage (246) definiert, die der gekrümmten Daumenauflage (244) gegenüberliegt.

4. Einführungsanordnung (1) nach einem der Ansprüche 1 bis 3, wobei das Paddel (38) eine Greifoberfläche (248) umfasst.

5. Einführungsanordnung (1) nach einem der Ansprüche 1 bis 4, wobei das Paddel (38) bemessen ist, um innerhalb des Aufnahmetrichters (20) aufgenommen zu werden.

6. Einführungsanordnung (1) nach einem der Ansprüche 1 bis 5, wobei das Paddel (38) ein Anschlussstück (42) umfasst, das mit dem Aufnahmetrichter (20) verriegelbar in Eingriff bringbar ist, um das Stilett (30) an dem Platzierungskatheter (10) entfernbar zu befestigen, optional wobei das Anschlussstück (42) mit einer Innenoberfläche des Aufnahmetrichters (20) in Eingriff bringbar ist.

7. Einführungsanordnung (1) nach Anspruch 6, wobei:
eines oder beide von dem Anschlussstück (42) und dem Aufnahmetrichter (20) mindestens einen Clip umfasst, der an dem anderen von dem Anschlussstück (42) und dem Aufnahmetrichter (20) befestigbar ist;
und/oder
das Anschlussstück (42) einen umlaufenden Grat definiert, und wobei der Aufnahmetrichter (20) einen umlaufenden Kanal definiert, der bemessen ist, um den umlaufenden Grat passend in Eingriff zu nehmen.

8. Einführungsanordnung (1) nach Anspruch 6 oder 7, wobei das Anschlussstück (42) konfiguriert ist, um eine hörbare und/oder taktile Rückmeldung während des Eingriffs mit dem Aufnahmetrichter (20) zu erzeugen.

9. Einführungsanordnung (1) nach einem der Ansprüche 1 bis 8, wobei:
der Aufnahmetrichter (20) eines oder beide von einem ovalen Profil und einem ovalen Umfang aufweist; und/oder
der Aufnahmetrichter (20) sich asymmetrisch in einer Richtung quer zu dem Stilettkörper (32) erstreckt.

10. Einführungsanordnung (1) nach einem der Ansprüche 1 bis 9, wobei der Aufnahmetrichter (20) ein Trichterlumen definiert, das konfiguriert ist, um Schmiermittel von dem Aufnahmetrichter (20) zu dem Lumen (12) des Platzierungskatheters (10) zu übertragen, optional wobei der Aufnahmetrichter (20) bemessen ist, um mindestens 1 mL des Schmiermittels aufzunehmen.

11. Einführungsanordnung (1) nach einem der Ansprüche 1 bis 10, wobei der Platzierungskatheter (10) transparent oder lichtdurchlässig ist, um die Position der intravesikalen Vorrichtung (40) oder des Stiletts (30) innerhalb des Lumens (12) visuell zu bestimmen, und/oder wobei der Platzierungskatheter (10) Tiefenmarkierungen umfasst, die die Einführungstiefe und die Orientierung der Austrittsöffnung (16) anzeigen.

12. Einführungsanordnung (1) nach einem der Ansprüche 1 bis 11, wobei ein distales Katheterende (18) des Platzierungskatheters (10) (i) eine abgerundete atraumatische Spitze und/oder (ii) eine Coudé-Biegung umfasst, die optional mit einer Hauptachse des Rückholtrichters ausgerichtet ist, so dass eine Orientierung des Rückholtrichters eine Orientierung der Coudé-Biegung anzeigt.

13. Einführungsanordnung (1) nach einem der Ansprüche 1 bis 12, wobei die Austrittsöffnung (16) an dem distalen Ende (18) des Platzierungskatheters (10) angeordnet ist und an dem Lumen (12) ausgerichtet ist.

14. Einführungsanordnung (1) nach einem der Ansprüche 1 bis 13, wobei der Stilettkörper (32) ein Lumen definiert, das sich von dem proximalen Stilettende (34) zu dem distalen Stilettende (36) erstreckt.

15. Einführungsanordnung (1) nach einem der Ansprüche 1 bis 14, wobei das Paddel (238) umfasst:
einen zentralen Körperabschnitt (231), der das proximale Stilettende (34) umgibt;
eine Daumenauflage (244), die an einem proximalen Paddelende (233) definiert ist; und
ein Paar von Flügelabschnitten (235a, 235b), die sich seitlich von dem zentralen Körperabschnitt (231) zwischen der Daumenauflage (244) und einem distalen Paddelende (237) erstrecken,
optional wobei die Daumenauflage (244) eine konkave Oberfläche, die von dem zentralen Körper (231) weg weist, eine oval geformte Peripherie, oder sowohl eine konkave Oberfläche, die von dem zentralen Körper (321) weg weist, als auch eine oval geformte Peripherie aufweist.

## Revendications

1. Ensemble d'insertion (1) destiné au déploiement d'un dispositif intravésical (40) dans une vessie d'un patient, l'ensemble d'insertion (1) comprenant :
un cathéter de mise en place (10) définissant une lumière (12), la lumière (12) s'étendant d'une extrémité proximale de cathéter (14) à une ouverture de sortie (16) ;
un entonnoir de réception (20) attaché au cathéter de mise en place (10) au niveau de l'extrémité proximale de cathéter ; et
un stylet (30) reçu de façon amovible à travers l'entonnoir de réception (20) au sein de la lumière (12) du cathéter de mise en place (10), dans lequel le stylet (30) comprend (i) un corps de stylet (32) s'étendant entre une extrémité proximale de stylet (34) et une extrémité distale de stylet (36), le corps de stylet (32) étant dimensionné pour entraîner le dispositif intravésical (40) à travers la lumière (12) et l'ouverture de sortie (16) dans la vessie, et (ii) une palette (38) fixée au corps de stylet (32) autour de l'extrémité proximale de stylet (34), dans lequel la palette (38) peut venir en prise avec l'entonnoir de réception (20).

2. Ensemble d'insertion (1) selon la revendication 1, dans lequel la palette (38) est surmoulée autour du corps de stylet (33) au niveau de l'extrémité proximale de stylet (34), ou est solidaire du corps de stylet (34).

3. Ensemble d'insertion (1) selon la revendication 1 ou 2, dans lequel la palette (38) définit un repose-pouce incurvé (244) au niveau d'une extrémité proximale (34), et définit facultativement en outre un appui de doigt incurvé (246) opposé au repose-pouce incurvé (244).

4. Ensemble d'insertion (1) selon l'une quelconque des revendications 1 à 3, dans lequel la palette (38) comprend une surface de prise (248).

5. Ensemble d'insertion (1) selon l'une quelconque des revendications 1 à 4, dans lequel la palette (38) est dimensionnée pour être reçue au sein de l'entonnoir de réception (20).

6. Ensemble d'insertion (1) selon l'une quelconque des revendications 1 à 5, dans lequel la palette (38) comprend un raccord (42) pouvant venir en prise de façon verrouillable avec l'entonnoir de réception (20) pour attacher de façon amovible le stylet (30) au cathéter de mise en place (10), facultativement dans lequel le raccord (42) peut venir en prise avec une surface intérieure de l'entonnoir de réception (20).

7. Ensemble d'insertion (1) selon la revendication 6, dans lequel :
l'un et/ou l'autre du raccord (42) et de l'entonnoir de réception (20) comprennent au moins une attache pouvant être attachée à l'autre du raccord (42) et de l'entonnoir de réception (20) ;
et/ou
le raccord (42) définit une crête circonférentielle, et dans lequel l'entonnoir de réception (20) définit un canal circonférentiel dimensionné pour venir en prise par accouplement avec la crête circonférentielle.

8. Ensemble d'insertion (1) selon les revendications 6 ou 7, dans lequel le raccord (42) est conçu pour générer un retour audible et/ou tactile pendant la mise en prise avec l'entonnoir de réception (20).

9. Ensemble d'insertion (1) selon l'une quelconque des revendications 1 à 8, dans lequel :
l'entonnoir de réception (20) a l'un et/ou l'autre d'un profil ovale et d'un périmètre ovale ; et/ou
l'entonnoir de réception (20) s'étend asymétriquement dans une direction transversale au corps de stylet (32).

10. Ensemble d'insertion (1) selon l'une quelconque des revendications 1 à 9, dans lequel l'entonnoir de réception (20) définit une lumière d'entonnoir conçue pour transférer un lubrifiant de l'entonnoir de réception (20) à la lumière (12) du cathéter de mise en place (10), facultativement dans lequel l'entonnoir de réception (20) est dimensionné pour recevoir au moins 1 mL du lubrifiant.

11. Ensemble d'insertion (1) selon l'une quelconque des revendications 1 à 10, dans lequel le cathéter de mise en place (10) est transparent ou translucide pour déterminer visuellement la position du dispositif intravésical (40) ou du stylet (30) au sein de la lumière (12), et/ou dans lequel le cathéter de mise en place (10) comprend des marquages de profondeur indiquant une profondeur d'insertion et une orientation de l'ouverture de sortie (16).

12. Ensemble d'insertion (1) selon l'une quelconque des revendications 1 à 11, dans lequel une extrémité distale de cathéter (18) du cathéter de mise en place (10) comprend (i) une pointe atraumatique arrondie, et/ou (ii) un coude, qui est facultativement aligné avec un grand axe de l'entonnoir de récupération de telle sorte qu'une orientation de l'entonnoir de récupération indique une orientation du coude.

13. Ensemble d'insertion (1) selon l'une quelconque des revendications 1 à 12, dans lequel l'ouverture de sortie (16) est au niveau de l'extrémité distale (18) du cathéter de mise en place (10) et est alignée avec la lumière (12).

14. Ensemble d'insertion (1) selon l'une quelconque des revendications 1 à 13, dans lequel le corps de stylet (32) définit une lumière s'étendant de l'extrémité proximale de stylet (34) à l'extrémité distale de stylet (36).

15. Ensemble d'insertion (1) selon l'une quelconque des revendications 1 à 14, dans lequel la palette (238) comprend :
une partie centrale (231) de corps entourant l'extrémité proximale de stylet (34) ;
un repose-pouce (244) défini au niveau d'une extrémité proximale de palette (233) ; et
une paire de parties d'ailette (235a, 235b) s'étendant latéralement à partir de la partie centrale (231) de corps entre le repose-pouce (244) et une extrémité distale de palette (237),
facultativement dans lequel le repose-pouce (244) a une surface concave faisant face à l'écart du corps central (231), une périphérie en forme d'ovale, ou à la fois une surface concave faisant face à l'écart du corps central (321) et une périphérie en forme d'ovale.
